Europäisches Patentamt

European Patent Office

Office européen des brevets

(11) Publication number: **0 338 715**
**A1**

## EUROPEAN PATENT APPLICATION

(21) Application number: **89303554.3**

(22) Date of filing: **11.04.89**

(51) Int. Cl.4: **A61F 2/42**

(30) Priority: **12.04.88 GB 8808577**

(43) Date of publication of application:
**25.10.89 Bulletin 89/43**

(84) Designated Contracting States:
**ES**

(71) Applicant: **Seedhom, Bahaa Botros**
**30 Blenheim Terrace**
**Leeds West Yorkshire LS2 9HD(GB)**

(72) Inventor: **Seedhom, Bahaa Botros**
**30 Blenheim Terrace**
**Leeds West Yorkshire LS2 9HD(GB)**

(74) Representative: **Orr, William McLean et al**
**URQUHART-DYKES & LORD 5th Floor, Tower**
**House Merrion Way**
**Leeds West Yorkshire, LS2 8PA(GB)**

(54) · Prosthetic finger joint.

(57) There is disclosed a prosthetic finger joint (10) for implantation between two finger bone sections (11 and 12), and which comprises a stem (13) for introduction into a passage (14) formed in the first finger section (11), and a part spherical head (15) provided at one end of the stem (13) to permit pivotting of the second bone section (12) relative to the first bone section (11). The head (15) defines a hemispherical bearing surface (16) over which the finger section (12) can pivot through approximately 90°, and it has a planar seating face (17) which engages an inclined end face (18) of the first finger section (11) after implantation surgery. The seating face (17) is non-perpendicularly inclined to the general axis of the passage (14) in the first finger bone section (11), and the stem (13) projects non-perpendicularly from the seating face (17) to be received by the passage (14). A prosthetic socket section (23) is mounted in the second finger bone section (12), and comprises a cup (24) which slidably engages the outer surface (16) of the head (15), and an integral stem (25) which is introduced into a passage (26) formed in the second bone section (12). The stems (13 and 25) can be anchored in position using acrylic bone cement, or in some embodiments the anchoring in position may be cementless, by virtue of the construction of the stems.

FIG. 1

## PROSTHETIC FINGER JOINT

This invention relates to a prosthetic finger joint and is particularly, though not exclusively, concerned with a replacement metacarpophalangial (MCP) joint.

It is known to provide prosthetic finger joints which comprise a stem having a ball end, in which the stem is secured to a centre region of a planar face of the ball end and projects substantially perpendicularly therefrom.

To replace a damaged or deteriorated finger joint between two finger sections, or a MCP joint, it is necessary to remove the remnants of the natural material providing the pivot axis between the finger sections and to drill a passage in one of the finger sections into which the stem is introduced.

The ball ends of existing prosthetic finger joints are generally hemispherical, and the spherical outer face provides a bearing surface which permits pivoting of the other finger section relative thereto. However, given that the ball end is hemispherical and has its planar end face extending perpendicular to the stem, this limits the extent of relative pivotal movement which can take place.

The most popular MCP replacement is the Swanson silicone rubber prosthesis which is a one component prosthesis with two stems introduced into the bone cavities, without any fixation. It restores the joint from a cosmetic point of view, but not its function to any appreciable extent. Also, in the course of time, natural ligament material may be completely damaged by disease. Absence of ligaments renders a joint unstable.

The present invention has been developed primarily with a view to providing a prosthetic finger joint which enables a greater range of relative arcuate movement between two finger sections after implantation, and preferably to facilitate the adhesion of re-growing natural ligament material and other tissue around the prosthetic joint to stabilise the said joint.

According to the invention there is provided a prosthetic finger joint for implantation between two finger bone sections and which comprises a stem for introduction into a passage formed in a first of the finger bone sections, and a part spherical bearing provided at one end of the stem to permit pivoting of a second finger bone section relative to the first finger bone section, the bearing being arranged to seat on an inclined end face of the first finger section so as to be non-perpendicularly inclined to the general axis of the passage, and in which:
the part spherical bearing has a substantially planar seating face which is engageable with said inclined end face of the first finger section, and said stem projects non-perpendicularly from said planar seating face to be receivable by said passage.

Preferably, the bearing is formed by a hemisphere, and the stem is secured non-centrally of the planar seating face of the bearing.

The planar seating face of the bearing may be formed with one or more recesses into which boney ingrowth can take place, or alternatively may comprise an annular seating which surrounds a single cavity defined within the bearing and into which boney ingrowth can take place.

To promote ingrowth of natural ligament material, it is preferred that the prosthetic finger joint is provided with a prosthetic ligament for co-operation therewith upon implantation thereof. The prosthetic ligament may comprise a perforated prosthetic ligament which may be attached to the prosthetic finger joint, or to any suitable site on the natural finger material, and natural material may then grow through the ligament over a period of time.

One preferred material from which the prosthetic finger joint may be made comprises a suitable grade of implantable plastics material. Alternatively, the bearing may be of composite construction, comprising a core of plastics material and a shell of any suitable biocompatible metal or metals.

When the core is made of plastics material, conveniently the stem also is moulded of plastics material in one piece with the core.

The external surface of the stem may be formed with serrations to engage the wall of the passage, and thereby to resist rotation of the stem in the passage.

In an alternative arrangement, the outer surface of the stem may be provided with resiliently deformable fins which enable the stem to be press-fitted into the passage by deformation of the fins as necessary, but which fins then engage firmly with the wall of the passage to locate the stem therein against axial withdrawal and / or rotation. This preferred arrangement may be designed so as to be capable of being secured in position without the need for subsequent application of acrylic bone cement i.e. a cementless implantation.

To enable a tighter fit to be made in the passage in the first bone section, a piece of fabric may be wrapped around the fins on the stem, and such fabric will also permit boney or tissue ingrowth over a period of time in order to anchor the implanted joint in position.

To faciliate the pivoting of the second finger bone section relative to the implanted prosthetic joint, it is particularly preferred to provide a prosthetic socket section for introduction into a passage formed in the second bone section. This preferably comprises a cup which is slidably engageable with the outer periphery of the bearing, and which has a central stem projecting away from the base of the cup and suitable for introduction into the bone passage in the second bone section.

The prosthetic socket section is preferably made of implantable grade plastics material.

When, as is preferred, the bearing is formed by a hemisphere, this permits a full 90° arc of movement of the second finger section relative to the first finger section.

While one preferred material from which the prosthetic finger joint can be made is implantable grade plastics material, or biocompatible metals, such as stainless steel, implantable ceramic materials also may be used.

Embodiments of prosthetic finger joint according to the invention will now be described in detail, by way of example only, with reference to the accompanying drawings, in which:

Figure 1 is a diagrammatic side view of a first embodiment of prosthetic finger joint according to the invention and intended to be used as an implant to replace an existing MCP joint;

Figure 2 is a plan view corresponding to Figure 1;

Figure 3 is a side view of one practical construction of prosthetic finger joint according to the invention;

Figure 3a is a detailed view of part of the outer periphery of a bearing head of the joint shown in Figure 3;

Figure 4 is an end view of the practical construction shown in Figure 3;

Figure 5 is a side view of a practical construction of prosthetic socket section for use with the joint shown in Figures 3 and 4; and,

Figure 6 is a detailed schematic view and to a reduced scale of an alternative construction of stem for the finger joint section.

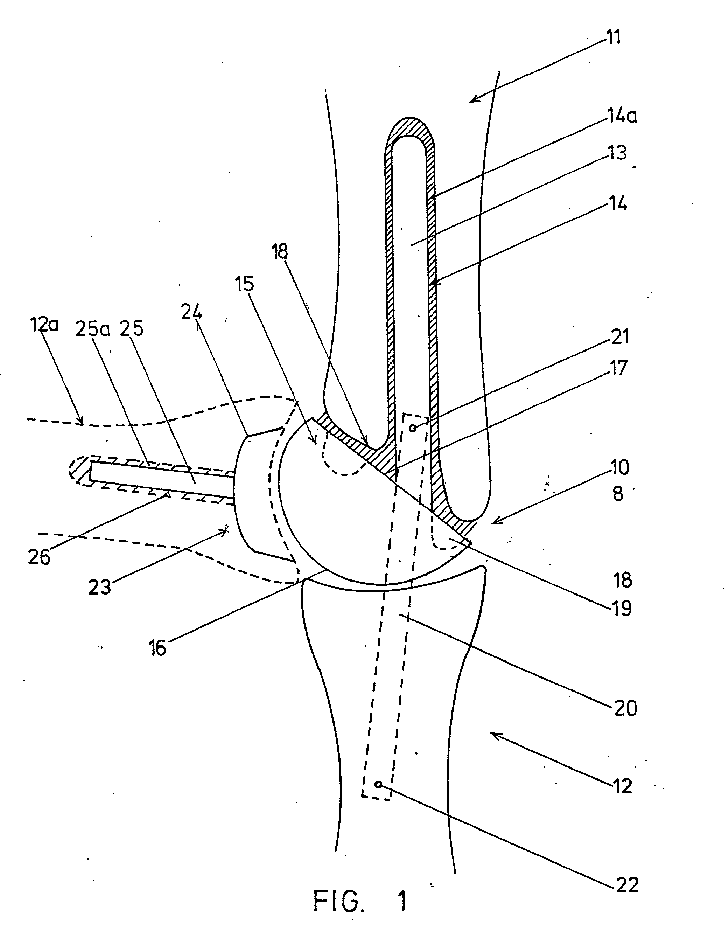

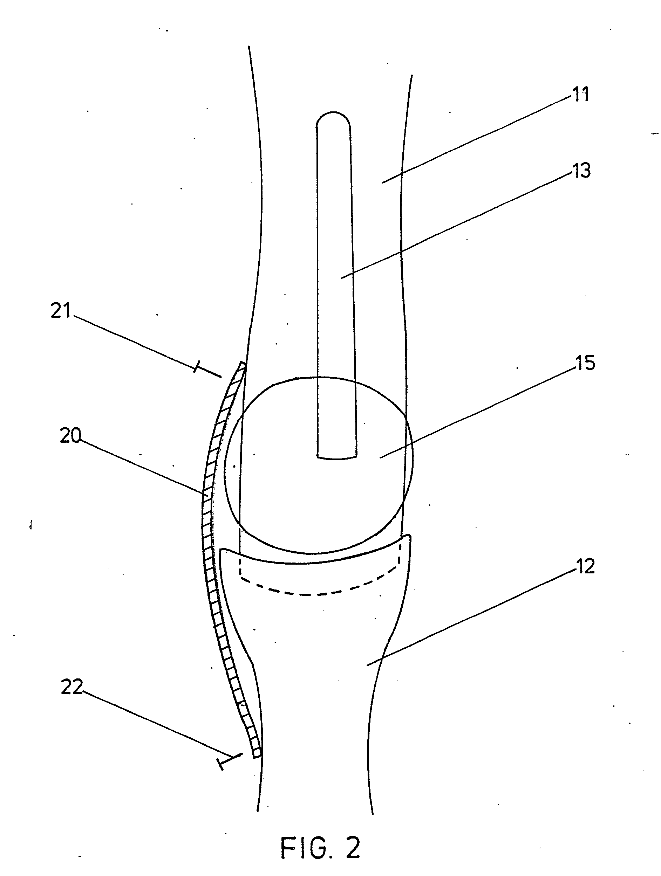

Referring now to Figures 1 and 2 of the drawings, a prosthetic finger joint according to the invention is designated generally by reference 10 and is intended for implantation between a first finger section 11 and a second finger section 12, which may be the finger bone sections adjacent to the MCP joint. The finger joint 10 comprises a stem 13 which is adapted for introduction into a passage 14 formed in the first finger section 11, and a part spherical head 15 provided at one end of the stem 13 to permit pivoting of the second bone section 12 relative to the first bone section 11.

The part spherical head 15 defines a hemispherical bearing surface 16 over which the finger section 12 can pivot through approximately 90° from the position shown in full lines in Figure 1 to the position shown in dashed outline. The head 15 also has a planar seating face 17 (which may be a generally annular surface) which engages an inclined end face 18 of the first finger section 11 after necessary implantation surgery. It will be noted from the side illustration of Figure 1 that the seating face 17 is non-perpendicularly inclined to the general axis of passage 14, and that the stem 13 projects non-perpendicularly from the seating face 17 to be received by the passage 14.

The stem 13 is therefore an off-set stem, in that it is non-perpendicularly inclined to seating surface 17, and also is non-centrally secured to the surface.

One or more cavities or recesses (shown by dotted outline 19 in Figure 1) may be provided in the seating face 17, to receive the entruded bone cement used for fixing the prosthesis. Alternatively, although not shown, the seating face 17 may be annular, and surrounds a single cavity formed in the head 15.

To promote natural ligament tissue ingrowth into and around the prosthetic finger joint, a perforated prosthetic ligament 20 may be attached to stem 13 and/or to the bearing 15, and is shown, for example, secured by pin 21 to the stem 13. After implantation surgery, the ligament 20 can be attached permanently to finger section 12, and this is shown diagramatically by the use of pin 22. In the course of time, natural tissue ingrowth will take place, which will be around, and through the prosthetic ligament, and into adhesion with the bone of the finger joint. A prosthetic ligament may be attached to either or each of the prosthetic finger joint components if desired, and may be secured in any desired manner. Alternatively, it may be secured to the bones of the finger joint.

The preferred material from which the prosthetic finger joint is manufactured is a biocompatible metal, such as stainless steel, or ceramic material, or part metal and part ceramic with the head being made of ceramics.

The illustrated embodiment of prosthetic finger joint will be particularly advantageous for use with a patient subject to the ulna deviation following failure of natural ligaments.

After implantation, the stem 13 will be anchored in position using acrylic bone cement 14a introduced with the passage 14.

To promote an easy sliding pivot connection between the adjacent finger sections 11 and 12, a prosthetic socket 23 is provided, which is shown for convenience within the section 12 at its pivoted position designated by reference 12a and shown in dashed outline in Figure 1. The socket 23 is made of suitable implantable plastics material, preferably ultra high molecular weight polyethylene (UHMWPE), and comprises a cup 24 which slidably engages the outer surface 16 of the head 15, and an integral stem 25 which is introduced into passage 26 formed in bone section 12 and then anchored in place using acrylic bone cement 25a.

The ligament 20 may be secured directly to the bones, by sutures or bone fragments. The shape of the ligament is preferably of open weave mesh with pockets, and / or dense sections as appropriate for fixation to both ends.

The stem 13 is preferably provided with a square cross-section, or is formed with "flats" so as to resist any tendancy for the prosthesis 10 to rotate in use, and the stem 13 to rotate in the acrylic cement 14a.

The head 15 (which is generally hemi-spherical), also may be formed with side flats to allow orientation during implantation, and / or to accommodate a larger spherical shape within a narrow finger joint.

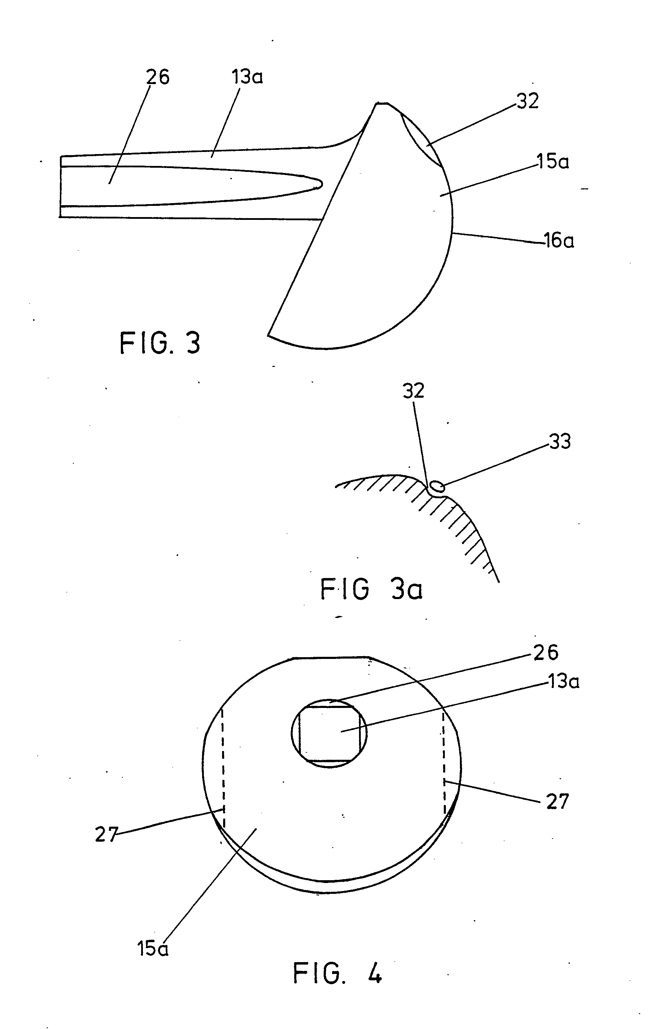

Figures 1 and 2 of the drawings show schematically one example of prosthetic finger joint and prosthetic socket section according to the invention. Practical constructions of the prosthetic finger joint and socket section are shown, by way of example only, in Figures 3 to 5, and parts corresponding with those already described with reference to Figures 1 and 2 are designated by the same reference numerals, but with the addition of the letter a.

It will be seen from Figures 3 and 4 that the stem 13a is provided with side flats 26 which will resist rotation of the stem 13a in the bone passage in the first finger bone section, and will allow easy introduction of acrylic bone cement. The head 15a may be provided with side cut-outs along the dashed lines 27, and which may be advantageous for certain types of finger joints requiring surgery e.g. to avoid impingement with an adjacent prosthetic joint and / or to avoid the resection of natural tissue which should be preserved.

In addition, the outer periphery of the bearing surface may have dents or grooves to allow sliding guidance of natural tendons, as shown in the detail of Figure 3a which shows a tendon 33 guided along a groove 32.

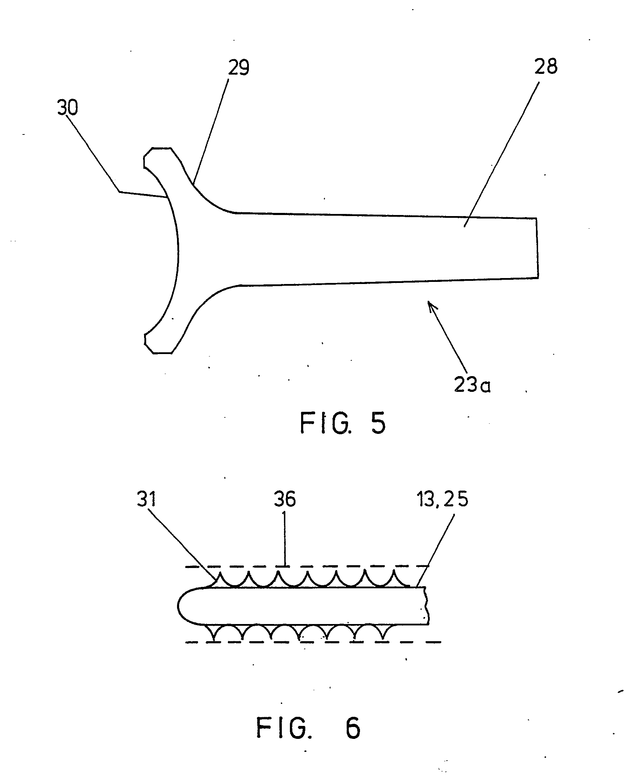

Figure 5 shows a prosthetic socket section 23a having a stem 28 and a cup 29 provided with a curved engaging surface 30 which can slide on the external surface 16a of head 15a of the prosthetic finger joint.

The illustrated embodiments are primarily intended to be anchored in position by the use of acrylic or other suitable bone cements, but a modification is shown in Figure 6, which enables a cementless joint to be implanted. Figure 6 is a detailed view of part of stem 13, or stem 25, and which is made of implantable grade plastics material, and has its external surface provided with resiliently deformable fins 31. These enable the stem (13, 25) to be press-fitted into its respective passage by deformation of the fins as necessary, but the fins then engage firmly with the wall of the passage to locate the stem therein against axial withdrawal and / or rotation.

Over a period of time, tissue and boney ingrowth can take place to anchor the stem in position, but to improve the anchoring effect, a piece of fabric, shown by dashed outline 32 in Figure 6, may be wrapped around the fins 31, and this enables a tighter fit to be made in the passage, but the fabric also permits boney or tissue ingrowth to take place over a period of time. The fabric covering may be formed by wrapping a fabric tape in spiral manner around the finned stem.

Some or all of the components of the prosthetic finger joint and prosthetic socket section may be made of implantable grade plastics material, or suitable biocompatible metal or metals, or ceramic material.

One preferred arrangement comprises moulding the stem 13 and a core of the head 15 in one piece of plastics material, and then applying a metal shell onto the core of the bearing head to form the outer bearing surface.

## Claims

1. A prosthetic finger joint for implantation between two finger bone sections and which comprises a stem for introduction into a passage formed in a first of the finger bone sections, and a part spherical bearing provided at one end of the stem to permit pivoting of a second finger bone section relative to the first finger bone section, the bearing being arranged to seat on an inclined end face of the first finger section so as to be non-perpendicularly inclined to the general axis of the passage, and in which:
the part spherical bearing has a substantially planar seating face which is engageable with said inclined end face of the first finger section, and said stem projects non-perpendicularly from said planar seating face to be receivable by said passage.

2. A prosthetic finger joint according to Claim 1, in which the bearing is formed by a hemisphere, and the stem is secured non-centrally of the planar seating face of the bearing.

3. A prosthetic finger joint according to Claim 1 or 2, in which the planar seating face of the bearing is formed with one or more recesses into which boney ingrowth can take place.

4. A prosthetic finger joint according to Claim 1 or 2, in which the planar seating face of the bearing comprises an annular seating which surrounds a single cavity defined within the bearing and into which boney ingrowth can take place.

5. A prosthetic finger joint according to any one of Claims 1 to 4, including a prosthetic ligament for co-operation with the prosthetic joint upon implantation thereof.

6. A prosthetic finger joint according to any one of Claims 1 to 5, in which the bearing is of composite construction comprising a core of plastics material and a shell of a biocompatible metal.

7. A prosthetic finger joint according to Claim 6, in which the stem is moulded of plastics in one piece with said core.

8. A prosthetic finger joint according to any one of Claims 1 to 7, in which the external surface of the stem is formed with serrations to engage the wall of the passage and thereby to resist rotation of the stem in the passage.

9. A prosthetic finger joint according to any one of Claims 1 to 7, in which the outer surface of the stem is provided with resiliently deformable fins which enable the stem to be press-fitted into the passage by deformation of the fins as necessary, but which fins then engage firmly with the wall of the passage to locate the stem therein against axial withdrawal and / or rotation.

10. A prosthetic finger joint according to Claim 9, including a piece of fabric which is wrapped around the fins on the stem to enable a tighter fit to be made in the passage, such fabric also permitting boney or tissue ingrowth over a period of time in order to anchor the implanted joint in position.

11. A prosthetic finger joint according to any one of Claims 1 to 10, including a prosthetic socket section for introduction into a passage formed in the second bone section.

12. A prosthetic finger joint according to Claim 11, in which the prosthetic socket section comprises a cup which is slidably engageable with the outer periphery of the bearing, and which has a central stem projecting away from the base of the cup and suitable for introduction into the passage in the second bone section.

13. A prosthetic finger joint according to any one of Claims 1 to 12, in which the outer periphery of the bearing is formed with a groove or dent along which a natural tendon can be guided.

14. A prosthetic finger joint according to Claim 2, in which the hemispherical bearing is formed with lateral cut-outs to reduce the transverse dimension of the joint.

15. A method of implanting a prosthetic finger joint between first and second finger bone sections, said prosthetic finger joint comprising a stem and a part spherical bearing provided at one end of the stem, and said bearing having a substantially planar seating face with said stem projecting non-perpendicularly from said seating face, and in which the method comprises:

forming a passage in the first finger bone section;
forming an inclined end face of the first finger section after implantation surgery; and,
introducing the stem of the prosthetic finger joint into said passage and seating the bearing via its planar seating face on said inclined end face so as to be non-perpendicularly inclined to the general axis of the passage.

16. A method according to Claim 15, in which a passage is formed in the second bone section, and a prosthetic socket section is introduced into said passage in the second bone section to co-operate with the bearing of the prosthetic finger joint.

17. A method according to Claim 16, in which the prosthetic socket section comprises a cup having a central stem projecting away from the base of the cup and which is introduced into the passage in the second bone section, said cup being slidably engageable with the outer periphery of the bearing of the prosthetic finger joint upon implantation.

FIG. 1

FIG. 2

26    13a

32

15a

16a

FIG. 3

32

33

FIG 3a

26

13a

27

27

15a

FIG. 4

FIG. 5

FIG. 6

European Patent Office

# PARTIAL EUROPEAN SEARCH REPORT

which under Rule 45 of the European Patent Convention shall be considered, for the purposes of subsequent proceedings, as the European search report

Application number

EP 89 30 3554

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-4 231 121 (F.M. LEWIS) | | A 61 F 2/42 |
| | * Whole document * | 1,2,11, 12 | |
| A | | 3-10, 13,14 | |
| | -- | | |
| Y | EP-A-0 042 808 (G. GAUTIER) | | |
| | * Page 4, lines 24-34; page 6, lines 1-15; figure 2 * | 1,2, 11,12 | |
| | -- | | |
| A | US-A-3 879 767 (J.A. STUBSTAD) | | |
| | * Column 2, line 63 - column 3, line 17; column 3, lines 31-50, 63-67 * | 3,4, 10 | |
| | -- | | |
| | ./. | | |

TECHNICAL FIELDS SEARCHED (Int. Cl.4)

A 61 F

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-14

Claims searched incompletely:

Claims not searched: 15-17

Reason for the limitation of the search:

Method for treatment of the human or animal body by surgery or therapy (see art. 52(4) of the European Patent Convention).

| Place of search | Date of completion of the search | Examiner |
|---|---|---|
| The Hague | 06-07-1989 | NICE |

| | DOCUMENTS CONSIDERED TO BE RELEVANT | | CLASSIFICATION OF THE APPLICATION (Int. Cl.4) |
|---|---|---|---|
| Category | Citation of document with indication, where appropriate, of relevant passages | Relevant to claim | |
| A | US-A-4 642 122 (A.D. STEFFEE)<br><br>* Column 1, line 47 - column 2, line 30; column 3, lines 19-22; figures 1,3 *<br><br>-- | 7-9, 14 | |
| A | WO-A-86 03 117 (L. HANSLIK et al.)<br><br>* Page 2, lines 8-17; page 2, line 27 - page 3, line 9 *<br><br>-- | 5 | |
| A | GB-A-1 573 608 (D. HOWSE & CO.)<br><br>* Page 1, lines 66-84; figures 1,2*<br><br>-- | | **TECHNICAL FIELDS SEARCHED (Int Cl.4)** |
| A | EP-A-0 053 633 (W. SCHNEIDER)<br><br>* Page 2, lines 36-44; figure 4 *<br><br>-- | 13 | |
| A | FR-A-1 550 013 (DOW CORNING)<br><br>* Whole document *<br><br>-- | | |
| P,X | FR-A-2 605 878 (LANDOS APPLICAT-IONS ORTHOPEDIQUES FRANCAISES)<br><br>* Page 2, line 35 - page 3, line 14; page 4, lines 23-26; page 5, lines 21-29; page 6, line 24 - page 7, line 4; page 7, line 35 - page 8, line 2; page 8, lines 9-14; figures 1,6 *<br><br>---------- | 1,6,7, 9,11 | |